# EUROPÄISCHE PATENTSCHRIFT

(11) **EP 4 394 455 B1**
(45) Veröffentlichungstag und Bekanntmachung des Hinweises auf die Patenterteilung: **11.11.2026**
(21) Anmeldenummer: 22217299.1
(22) Anmeldetag: 30.12.2022
(51) Int. Cl.: G01T 1/20, G01T 1/24

(54) **DETEKTORMODUL FÜR EINEN RÖNTGENDETEKTOR MIT EINEM FUNKMODUL**
DETECTOR MODULE FOR AN X-RAY DETECTOR WITH A RADIO MODULE
MODULE DÉTECTEUR POUR DÉTECTEUR DE RAYONS X COMPRENANT UN MODULE RADIO

(43) Veröffentlichungstag der Anmeldung: 03.07.2024
(73) Patentinhaber: Siemens Healthineers AG, 91301 Forchheim (DE)
(72) Erfinder: Dr. Hosemann, Michael, 91056 Erlangen (DE)
(74) Vertreter: Siemens Healthineers Patent Attorneys

(56) Entgegenhaltungen:
- DE-A1- 102011 013 057
- DE-A1- 102015 203 764
- US-A1- 2010 096 556
- US-A1- 2011 218 432
- US-B1- 9 606 245

## Beschreibung

Die Erfindung betrifft ein Detektormodul für einen Röntgendetektor aufweisend eine Sensoreinheit, zumindest eine Ausleseeinheit und zumindest eine in Stapelanordnung zur Sensoreinheit angeordnete Elektronikeinheit und umfassend ein Funkmodul mit einem Funkschaltkreis, wobei der Funkschaltkreis des Funkmoduls zumindest teilweise in ein Einbettungsmaterial der Elektronikeinheit eingebettet ist. Die Erfindung betrifft weiterhin einen Röntgendetektor und ein medizinisches Bildgebungsgerät umfassend ein solches Detektormodul.

Unabhängig vom grammatikalischen Geschlecht eines bestimmten Begriffes sind im Folgenden Personen mit männlicher, weiblicher oder anderer Geschlechteridentität mit umfasst.

Röntgendetektoren finden in vielen bildgebenden Anwendungen Einsatz. So werden Röntgendetektoren beispielsweise in Computertomographie-Systemen (CT-System) in der medizinischen Bildgebung genutzt, um ein tomographisches Röntgenbild eines Untersuchungsbereiches eines Untersuchungsobjekts zu erzeugen.

CT-Systeme sind allgemein bekannt. Es ist auch allgemein bekannt, dass im rotierenden Teil eines CT-Systems große Datenmengen anfallen, die an den stationären Part übertragen werden müssen, insbesondere müssen die in einem oder in mehreren die Systemachse des CT-Systems umlaufenden Detektoren anfallenden Detektordaten zeitnah zur Auswertung übertragen werden.

Derzeit findet die Datenübertragung üblicherweise mit Hilfe von Schleifringsystemen statt, die über eine kapazitive Kopplung zwischen dem rotierenden und dem stationären Part eine Datenübertragungsstrecke aufbauen. Diese Technologie kann jedoch an ihre Grenzen stoßen, wenn die zu übertragende Datenmenge in der Zukunft mit der Weiterentwicklung der Detektoren weiter steigt. So sind beispielsweise die Datenübertragungsanforderungen von zählenden Detektoren, die eine wesentlich feinere Pixelierung aufweisen und zudem auch noch je Pixel mehrere Energieschwellen besitzen, die nochmals die zu übertragende Datenmenge erhöhen, im Vergleich zu konventionellen Detektoren stark gestiegen. Weiterhin kann ein solches Schleifringsystem durch Abnutzung und Verschmutzung anfällig für Leistungseinbußen sein.

Grundsätzlich sind Überlegungen bekannt für die Übertragung der Detektordaten zwischen einem rotierenden Detektor und einem stationären Teil des CT-Systems oder einem Empfänger im Anwendungsraum Funkübertragungsstrecken, d.h. mittels eines Verfahrens der drahtlosen Datenübertragung, anstelle von Schleifringsystemen zu verwenden. Neben Vorteilen bei der Übertragung großer Datenmengen, kann die Umsetzung der Datenübertragung mittels Funkübertragung im Vergleich zur Umsetzung mittels Schleifring Kosten bei Wartung und Erneuerung von Systemen reduzieren. Bei der Bereitstellung eines Detektors, welches eine solche Funkübertragung von Detektordaten ermöglicht, ist dabei stets eine kosteneffiziente und leicht handzuhabende Umsetzung erstrebenswert.

Detektormodule für einen Röntgendetektor mit drahtloser Datenübertragung sind bekannt aus DE102011013057A1 und US9606245B1. Aufgabe der Erfindung ist es daher ein verbessertes Detektormodul für einen Röntgendetektor vorzuschlagen, welches eine Datenübertragung mittels eines Verfahrens der drahtlosen Datenübertragung ermöglicht.

Die Aufgabe wird gelöst durch die Merkmale der unabhängigen Patentansprüche. Weitere vorteilhafte und teils für sich erfinderische Ausführungsformen und Weiterbildungen der Erfindung sind in den Unteransprüchen und der nachfolgenden Beschreibung dargelegt.

Die Erfindung betrifft ein Detektormodul für einen Röntgendetektor aufweisend
- eine Sensoreinheit, ausgebildet zur Konvertierung von eintreffender Röntgenstrahlung in elektrische Signale, und aufweisend eine Vielzahl an Pixelelementen,
- zumindest eine Ausleseeinheit in Stapelanordnung mit der Sensoreinheit, ausgebildet zur Auslese der elektrischen Signale von der Sensoreinheit, wobei jedes Pixelelement mit einem zugeordneten Auswertepixelelement der Ausleseeinheit verbunden ist,
- ein Funkmodul mit einem Funkschaltkreis, welches ausgebildet ist, die ausgelesenen elektrischen Signale mittels eines Verfahrens der drahtlosen Datenübertragung zu versenden, und
- eine in Stapelanordnung zur Sensoreinheit angeordnete Elektronikeinheit aufweisend zumindest eine elektrisch leitende Verbindung zur Weiterleitung der ausgelesenen elektrischen Signale von der zumindest einen Ausleseeinheit an das Funkmodul, wobei der Funkschaltkreis des Funkmoduls zumindest teilweise in ein Einbettungsmaterial der Elektronikeinheit eingebettet ist, wobei die Elektronikeinheit einen Flächenbereich aufweist, welcher in einer Richtung senkrecht zur Stapelrichtung über die flächige Ausdehnung der Sensoreinheit übersteht, und wobei das Funkmodul in dem überstehenden Flächenbereich angeordnet ist.

Die Sensoreinheit kann dabei ein direkt-konvertierendes oder ein indirekt-konvertierendes Konvertermaterial umfassen. Die Röntgenstrahlung bzw. die Röntgenphotonen können in direkt-konvertierenden Sensoreinheiten durch ein geeignetes Konvertermaterial in elektrische Signale umgewandelt werden. Als Konvertermaterial können beispielsweise CdTe, CZT, CdZnTeSe, CdTeSe, CdMnTe, InP, TlBr2, HgI2, GaAs oder andere verwendet werden. Die Röntgenstrahlung bzw. die Photonen können in indirekt-konvertierenden Sensoreinheiten durch ein geeignetes Konvertermaterial in Licht und mittels optisch gekoppelten Photodioden, insbesondere ein Photodiodenarray, in elektrische Pulse umgewandelt werden. Als Konvertermaterial werden häufig Szintillatoren, beispielsweise GOS (Gd2O2S), CsJ, YGO oder LuTAG, eingesetzt. Die Sensoreinheit kann insbesondere eine flächige Ausdehnung entlang zweier senkrecht zu der Stapelrichtung der Stapelanordnung verlaufenden Richtungen aufweisen. Sie kann ein einstückiges Konverterelement aufweisen oder aus einer Mehrzahl, welche entlang der flächigen Ausdehnung nebeneinander positioniert sind, zusammengesetzt sein.

Das Strahlungsdetektormodul umfasst bei beiden Typen jeweils eine Vielzahl von Pixelelementen, also kleinsten flächigen Bereichen, die eigenständig ausgelesen werden können. Um ausgelesen zu werden, ist jedes Pixel mit einem zugeordneten Auswertepixelelement einer Ausleseeinheit verbunden. Es kann eine Ausleseeinheit aber auch eine Mehrzahl an Ausleseeinheiten im Detektormodul, welche der Sensoreinheit zugeordnet sind, vorliegen. Eine Ausleseeinheit dient allgemein der Digitalisierung der elektrischen Signale, welche Sensoreinheit eingespeist werden. Eine Ausleseeinheit kann beispielsweise als ASIC (application specific integrated circuit) implementiert sein.

Die Sensoreinheit liegt erfindungsgemäß in Stapelanordnung mit der Elektronikeinheit vor. Die Stapelrichtung der Stapelanordnung verläuft im Wesentlichen parallel zu einer Strahleneinfallsrichtung von Röntgenstrahlung zur Belichtung eines Röntgendetektors, wenn das Detektormodul eingesetzt wird. Die Sensoreinheit ist dabei in der Stapelanordnung der einfallenden Strahlung zugewandt, wobei die Elektronikeinheit dieser in Strahleneinfallsrichtung nachgeordnet ist. Die Elektronikeinheit weist in der Stapelanordnung eine flächige Ausdehnung auf, welche sich parallel zur flächigen Ausdehnung der Sensoreinheit erstreckt. Mehrere Sensoreinheiten können einer gemeinsamen Elektronikeinheit zugeordnet sein. Die Elektronikeinheit erstreckt sich über die flächige Erstreckung der Sensoreinheit oder der Sensoreinheiten hinaus und weist eine größere flächige Ausdehnung auf. Es ist dabei nicht ausgeschlossen, dass zwischen der Elektronikeinheit und der Sensoreinheit weitere Elemente in Stapelanordnung vorliegen können. Weiterhin ist nicht ausgeschlossen, dass die Stapelanordnung weitere Elemente umfasst, welche entlang der Stapelrichtung vor oder nachgelagert und in Stapelanordnung vorliegen können. Beispielsweise kann dies ein Streustrahlengitter, einen Modulträger, einen Kühlkörper oder weitere Einheiten, welche für den Betrieb des Detektormoduls notwendig sind, betreffen.

Die zumindest eine Ausleseeinheit oder die Mehrzahl an Ausleseeinheiten liegt bzw. liegen ebenfalls in Stapelanordnung mit der Sensoreinheit vor. Die zumindest eine Ausleseeinheit weist oder die mehreren Ausleseeinheiten weisen dann ebenfalls eine flächige Ausdehnung auf, welche sich in der Stapelanordnung parallel zu der flächigen Ausdehnung der Sensoreinheit erstreckt.

Die zumindest eine Ausleseeinheit kann oder die mehreren Ausleseeinheiten können direkt anschließend an die Sensoreinheit angeordnet und mit dieser, beispielsweise über sogenannte bump bonds oder eine Leitklebeverbindung, für eine Signalübertragung verbunden sein. Sie kann bzw. können jedoch auch über ein oder mehrere weitere Elemente, beispielsweise eine dazwischen angeordnete Elektronikeinheit, hinweg mittels entsprechend ausgebildeten elektrisch leitenden Verbindungen mit der Sensoreinheit verbunden sein. Weiterhin, gemäß einer Ausbildungsvariante, kann die zumindest eine Ausleseeinheit auch in das Einbettungsmaterial der Elektronikeinheit zumindest teilweise eingebettet sein.

Die Elektronikeinheit umfasst in der Regel ein nicht leitendes Einbettungsmaterial, insbesondere ein Polymer, beispielsweise ein Plastikmaterial oder ein Epoxidharz, in welches zumindest der Funkschaltkreis zumindest teilweise eingebettet ist und eine oder mehrere elektrisch leitende Verbindungen, welche ebenfalls eingebettet in das Material oder auf einer Oberfläche der Elektronikeinheit ausgebildet sind.

Zumindest eine elektrisch leitende Verbindung der Elektronikeinheit dient dazu, die von der zumindest einen Ausleseeinheit ausgelesenen und ggf. von der Ausleseeinheit vorverarbeiteten elektrischen Signale, im Folgenden auch Detektordaten genannt, an das erfindungsgemäße Funkmodul weiterzuleiten. Es können dafür insbesondere auch eine Mehrzahl an elektrisch leitenden Verbindungen vorgesehen sein. Die zumindest eine elektrisch leitende Verbindung zur Signalübertragung zwischen der zumindest einen Ausleseeinheit und dem Funkmodul kann in das Einbettungsmaterial der Elektronikeinheit eingebettet oder auf dem Einbettungsmaterial der Elektronikeinheit aufgebracht sein. Eine Verdrahtung kann beispielsweise lithographisch, mittels einer Sputtertechnik, eines Dampfphasen-Abscheidungsverfahrens oder mittels eines Verfahrens des Leitungsdrucks auf eine Oberfläche des Einbettungsmaterial der Elektronikeinheit aufgebracht sein. Weiterhin können Durchkontaktierungen vorgesehen sein, welche elektrisch leitende Verbindungen durch das Einbettungsmaterial hindurch ermöglichen.

Je nach Anordnung der Elektronikeinheit oder der Ausleseeinheit in der Stapelanordnung kann die Elektronikeinheit elektrisch leitende Verbindungen zwischen der Sensoreinheit und der Ausleseeinheit aufweisen. Weiterhin können auch weitere elektrisch leitende Verbindungen, beispielsweise für die Zuführung von Steuerungssignalen zu der oder den Ausleseeinheiten oder dem Funkmodul oder für die Zuführung einer Betriebsspannung aufweisen. Die Elektronikeinheit kann auch weitere elektronische Bauteile umfassen, welche auf oder in ihr angeordnet sind und welche für den Betrieb des Detektormoduls vorgesehen sind.

Die Elektronikeinheit kann die Stabilität und damit die Handhabbarkeit der Stapelanordnung vorteilhaft erhöhen. Weiterhin kann die Elektronikeinheit eine Umverdrahtung von Kontaktstellen, beispielsweise von der Sensoreinheit zu der oder den Ausleseeinheiten, und damit vorteilhaft unterschiedliche ggf. kosteneffizientere Baugrößen oder auch die Zusammenfassung von Daten von mehreren Ausleseeinheiten vor einer Weiterleitung zu dem Funkmodul ermöglichen.

Das Funkmodul ist ausgebildet, die Detektordaten mittels eines Verfahrens der drahtlosen Datenübertragung, d.h. mittels einer Funktechnik zu versenden. Das Funkmodul als Sender arbeitet dabei in der Regel mit einem außerhalb des Röntgendetektors platzierten weiteren Funkmodul als Empfänger zusammen, so dass die Detektordaten von dem Detektormodul zu dem außerhalb des Röntgendetektors platzierten, weiteren Empfänger-Funkmodul übertragen werden können. Es kann auch Ausbildungen geben, wobei das Funkmodul des Detektormoduls außerdem als Empfänger ausgebildet sein kann, um Funksignale, beispielsweise umfassend Ansteuerungssignale für das Detektormodul, von einem Sender, welcher außerhalb des Röntgendetektors platziert ist, zu empfangen. Derart könnte auch eine Übermittlung von Ansteuerungssignalen für das Detektormodul vorteilhaft mittels drahtloser Datenübertragung durchgeführt werden. Vorteilhaft könnten weitere Datenübertragungswege für die Ansteuerung über eine Schleifringkonstruktion vermieden werden und Verkabelungsaufwand reduziert werden. Der Sender kann dann vom gleichen Funkmodul umfasst sein, welches auch für den Empfang der Detektordaten vorgesehen ist.

Es kann auch vorgesehen sein, dass ein Detektormodul mehrere Funkmodule umfasst, wobei dann die Detektordaten für die drahtlose Datenübertragung auf die mehreren Funkmodule aufgeteilt werden.

Das Funkmodul weist zumindest einen Funkschaltkreis und eine Funkantenne auf. Bezüglich der konkreten Ausgestaltung des Funkmodul wird vorgeschlagen, dass dieses mindestens einen Funkschaltkreis in Form eines integrierten Schaltkreises (engl. integrated circuit, IC), auch Festkörperschaltkreis genannt, umfasst. Insbesondere kann dieser als ASIC (application-specific integrated circuit, dt: anwendungsspezifische integrierte Schaltung) ausgebildet sein. Die Funkantenne kann in vorteilhaften Ausbildungen insbesondere als Antennenarray ausgebildet sein.

Der Funkschaltkreis ist zusammen mit der Funkantenne ausgebildet die Detektordaten, welche von der oder den Ausleseeinheiten an das Funkmodul weitergeleitet werden, mittels eines Verfahrens der drahtlosen Datenübertragung zu versenden, d.h. insbesondere an eine Empfängereinheit zu übertragen. Das Verfahren der drahtlosen Datenübertragung bzw. auch die Umsetzung von Antennen ist insbesondere derart gewählt, dass es für die Datenübertragung der Detektordaten geeignet ist und ist insbesondere auch auf die konkrete Ausbildung und den konkreten Einsatz des Detektormoduls anzupassen. So kann für einen Röntgendetektor in einem CT-System andere Anforderungen, beispielsweise hinsichtlich Datenmenge und vorhandenen Störsignale, vorliegen als in einem anderen medizinischen Gerät. Ebenso ist die Positionierung des Senders relativ zum Empfänger zu berücksichtigen. Eine Anordnung eines Empfängers im stationären Teil eines CT-Systems, insbesondere in direkter Nähe zum Rotor, ermöglicht beispielsweise durch einen deutlich geringeren Abstand zwischen einem Funkmodul des Detektormoduls und dem Empfänger eine andere Umsetzung als das Vorsehen eines Empfängers an einem Standort außerhalb des CT-Geräts, beispielsweise separat im Anwendungsraum. Ein Verfahren der drahtlosen Datenübertragung kann beispielsweise ein WLAN (wireless Local Area Network, dt.: drahtloses lokales Netzwerk)- Standard nutzen, welche in der Regel Frequenzbänder im Bereich von 2,4GHz oder 5GHz nutzen. Es können jedoch auch andere Umsetzungen vorgesehen sein, insbesondere auch solche die mit auf geringere Distanzen zwischen Sender und Empfänger optimiert sind, wenn die Anordnung im Anwendungsgerät, beispielsweise im CT-System, dies erlaubt.

Erfindungsgemäß ist der Funkschaltkreis des Funkmoduls zumindest teilweise in das Einbettungsmaterial der Elektronikeinheit eingebettet. Die Elektronikeinheit umfasst wie zuvor bereits beschrieben folglich zumindest den in das Einbettungsmaterial der Elektronikeinheit eingebetteten Funkschaltkreis sowie entsprechende elektrisch leitende Verbindungen und ggf. Kontakte, welche in sie integriert oder auf eine Oberfläche aufgebracht sind, um eine Signalübertragung zum Funkschaltkreis zu ermöglichen. Dies kann vorteilhaft die Robustheit des Bauteils erhöhen und den Schutz vor Beschädigungen der Bauteile erhöhen. Vorteilhaft kann außerdem auf Herstellungsverfahren für die Elektronikeinheit mit dem Funkschaltkreis zurückgegriffen werden, welche auf der Verwendung von sogenannten "bare dies" (dt.: nackte Chips) für den Funkschaltkreis zurückgreift, was insbesondere kostengünstig sein kann.

Weiterhin kann damit vorteilhaft ein sehr kompaktes Bauteil mit geringen Abmessungen erreicht werden, welches eine Montage des Detektormoduls erleichtern kann. Der Funkschaltkreis kann insbesondere zumindest an drei Seiten zumindest teilweise eingebettet sein. Er kann auch vollständig in das Einbettungsmaterial eingebettet sein.

Die Elektronikeinheit kann insbesondere mit sogenannten Wafer-Level-Packaging-Methoden, bei dem Chips in Polymer-Verkapselungen eingebettet werden, bevorzugt einer Methode des Fan-out Wafer-Level Packaging, oder Panel-Level Packaging-Methoden hergestellt sein. Vorteilhaft können hier in kostengünstiger Weise und parallelisiert in größerer Anzahl Elektronikeinheiten mit bereits integrierten Funkschaltkreisen bereitgestellt werden. Die Elektronikeinheit bildet in diesem Fall mithin ein Bauteil, das den Funkschaltkreis, das diesen umgebende Gehäuse und ggf. elektrisch leitende Verbindungen für die Kontaktierung umfasst. Panel-Level Packaging-Methoden ermöglichen aufgrund der größeren Anzahl an parallel verarbeitbarer Chips eine weitere Steigerung der Produktivität und daraus resultierenden geringeren Package-Kosten. Die Methoden bieten außerdem vorteilhaft ein sehr dünnes Package, einen geringen thermischen Widerstand, geringe Störungen und Induktivitäten aufgrund kurzer elektrischer Verbindungen. Eine alternative Variante umfasst PCB (Printed Circuit Board)-Embedding-Methoden, bei dem die Chips in Leiterplattenmaterial eingebettet werden.

In alternativen, hier nicht beanspruchten Ausbildungen eines Detektormoduls kann auch vorgesehen sein, dass der Funkschaltkreis lediglich auf der Elektronikeinheit angeordnet ist.

Vorteilhaft ist das Detektormodulen unmittelbar mit einer Funkeinheit auszustatten, so dass die Detektordaten in unmittelbarer Nähe zu ihrem Entstehungsort an die Funkeinheit übergeben und die Übertragung stattfinden kann. Vorteilhaft sind die Übertragungswege und Zwischenschritte kurz. Aufgrund der Integration des Funkmoduls in die Stapelanordnung können sich vorteilhaft notwendige Bauteile und Verkabelungsaufwand reduzieren. Ein weiterer Vorteil bietet sich auch dadurch, dass die Funkübertragungstechnologie eine weit verbreitete Verwendung auch außerhalb von Röntgendetektoren oder der Computertomographie besitzt und entsprechend auch wesentlich kostengünstigere Bauteile bietet als beispielsweise bei Nutzung der Schleifringtechnologie. Vorteilhaft wird insgesamt in Form der erfindungsgemäßen Stapelanordnung ein äußerst robustes Bauteil bereitgestellt, dass eine einfache Handhabbarkeit und damit eine kosteneffiziente Montage erlaubt.

Gemäß einer vorteilhaften Ausbildungsvariante ist die zumindest eine Ausleseeinheit ebenso wie der Funkschaltkreis in das Einbettungsmaterial der Elektronikeinheit zumindest teilweise, insbesondere zumindest teilweise von drei Seiten oder vollständig, eingebettet. Vorteilhaft kann dies den Schutz vor Beschädigungen der Ausleseeinheiten erhöhen, ebenso kann hiermit ein äußerst kompaktes Bauteil bereitgestellt werden, welches die zusätzlichen Raumverbrauch durch eine separat aufgebrachte Ausleseeinheit vermeidet. Auch ist es möglich, wie beim Funkschaltkreis auf Herstellungsverfahren zurückzugreifen, welche eine hohe Parallelisierung in der Bereitstellung oder welche beispielsweise auf der Verwendung von sogenannten "bare dies" (nackten Chips) für den Schaltkreis zurückgreift, was insbesondere kostengünstig sein kann.

Erfindungsgemäß weist die Elektronikeinheit einen Flächenbereich auf, welcher in einer Richtung senkrecht zur Stapelrichtung über die flächige Ausdehnung der Sensoreinheit übersteht, wobei das Funkmodul in dem überstehenden Flächenbereich angeordnet ist. Dadurch wird eine drahtlose Datenübertragung nicht durch die Sensoreinheit oder andere Komponenten des Detektormoduls gestört. In vorteilhafter Weise kann außerdem vermieden werden, dass der Funkschaltkreis zu einem Wärmeeintrag in die Sensoreinheit führt, was zu Einbußen bei einer Bildqualität führen könnte.

Gemäß einer vorteilhaften Ausbildung weist das Funkmodul zumindest eine Funkantenne auf, welche einen auf einer Oberfläche des Einbettungsmaterials der Elektronikeinheit oder auf einer Oberfläche des Funkschaltkreises aufgebrachten Draht umfasst. Dieser kann beispielsweise lithographisch, mittels einer Sputtertechnik oder mittels eines Verfahrens des Leitungsdrucks aufgebracht sein. Vorteilhaft erlaubt eine lithographische Aufbringung eine besonders präzise und hochfeine Strukturierung. Insbesondere kann das Vorsehen der Antenne bereits bei Herstellung der Elektronikeinheit, d.h. beispielsweise als Teil eines Packaging-Verfahrens aufgebracht werden. Vorteilhaft kann eine kostengünstige Bereitstellung ermöglicht sein, wobei außerdem auf zusätzliche Komponenten verzichtet werden kann. Insbesondere stellt dies eine vorteilhaft robuste und kompakte Umsetzung dar. Vorteilhaft wird mit der Elektronikeinheit ein Bauteil bereitgestellt, in welchem die Komponenten für eine drahtlose Datenübertragung und deren Kontaktierung integriert vorliegen.

Gemäß einer weiteren Ausbildungsvariante kann das Detektormodul weiterhin einen separaten Steuerungsschaltkreis aufweisen, welcher ausgebildet ist, die zumindest eine Ausleseeinheit und/oder das Funkmodul anzusteuern. In einer alternativen Ausbildung sind der Funkschaltkreis und der Steuerschaltkreis, welche eine Ansteuerung des Funkschaltkreises erlaubt, ein integrales Bauteil. Dies setzt jedoch ein darauf zielgerichtetes Design und damit eine anwendungsspezifische Ausbildung voraus. Indem der Steuerungsschaltkreis und der Funkschaltkreis separat ausgebildet ist kann erleichtert auf kostengünstigere, einfach zu beziehende Standard-Bauteile zurückgegriffen werden. Weiterhin kann der Steuerschaltkreis auch ausgebildet sein, die Ausleseeinheiten anzusteuern. Dies kann umfassen, lediglich den Ausleseprozess anzusteuern. Es kann jedoch auch umfassen, beispielsweise eine Konfiguration der Ausleseeinheiten für den Messprozess anzusteuern. Der Steuerschaltkreis kann ebenso als ASIC ausgebildet sein. In vorteilhaft günstigen Ausbildungsvarianten kann der Steuerschaltkreis als FPGA (field programmable gate array, dt.: im Feld programmierbare (Logik-)Gatter-Anordnung). Dies kann auch eine flexiblere Anpassung des Steuerschaltkreises erlauben, da hier eine wiederholte Programmierung der Funktionalitäten des Steuerschaltkreises möglich ist.

Gemäß einer Ausbildungsvariante davon ist auch der Steuerungsschaltkreis zumindest teilweise in das Einbettungsmaterial der Elektronikeinheit eingebettet, so dass vorteilhaft ein kompakter Aufbau bereitgestellt ist. Die Vorteile der Einbettung stellen sich im Wesentlichen gleich der Vorteile der Einbettung des Funkschaltkreises oder einer Ausleseeinheit dar. Vorteilhaft wird ein integrales, kompaktes und robustes Bauteil bereitgestellt.

Gemäß einer Ausbildungsvariante ist die Elektronikeinheit in der Stapelanordnung insbesondere zwischen der Sensoreinheit und der zumindest einen Ausleseeinheit angeordnet. Vorteilhaft ermöglicht dies die Umverdrahtung einer größeren Sensoreinheit auf einen oder mehrere Ausleseeinheiten mit geringerer flächigen Ausdehnung, welche kostengünstiger bereitstellbar sind. Gleiches kann auch vorteilhaft erreicht werden, wenn die Ausleseeinheit in die Elektronikeinheit eingebettet ist und eine entsprechende Umverdrahtungslage auf oder in der Elektronikeinheit vorgesehen ist.

Die Sensoreinheit kann, wie bereits zuvor beschrieben, je nach Ausbildungsvariante beispielsweise als direktkonvertierende Sensoreinheit ausgebildet sein oder ein Szintillatorelement und zumindest ein Photodiodenarray umfassen. Insbesondere bei der Anwendung von direktkonvertierenden Sensoreinheiten fallen in der Regel eine sehr große Menge an Daten an, welche vorteilhaft mittels einer Umsetzung, welche eine drahtlose Datenübertragung mittels eines Funkmoduls umfasst, verbessert mit hoher Datenrate übertragen werden können. Jedoch kann eine derartige Umsetzung auch mit Szintillator-basierten Umsetzungen vorteilhaft sein, weil die Nachteile einer Schleifringbasierten Umsetzung vermieden werden können.

Die Erfindung betrifft weiterhin einen Röntgendetektor umfassend eine Mehrzahl an Detektormodulen nach einem der vorangehenden Ausbildungsvarianten. Die Mehrzahl an Detektormodulen kann insbesondere nebeneinander in dem Röntgendetektor angeordnet sein, so dass die Sensoreinheiten insgesamt als größere Detektionsfläche zusammenwirken.

Vorteilhaft weist jedes Detektormodul ein Funkmodul auf, so dass die in einem Detektormodul entstehenden Detektordaten unmittelbar an die jeweilige in diesem Detektormodul vorliegende Funkeinheit übergeben und ohne weitere Zwischenschritte und -wege direkt übertragen werden können. Auf diese Weise wird auch eine Übertragung der Daten über längere Datenübertragungsstrecken hinweg zu einer Sendeeinheit vermieden. Weiterhin kann eine parallele Datenübertragung der Detektordaten von den Modulen eine insgesamt besonders hohe Datenübertragungsrate ermöglichen. Weiterhin erlaubt dies vorteilhaft Detektoren mit unterschiedlicher Modulzahl ohne wesentlichen Aufwand in der Entwicklung elektronischer Hardware herzustellen.

Es wäre auch denkbar, dass ein erfindungsgemäßer Röntgendetektor Detektormodule mit einem Funkmodul und solche ohne ein Funkmodul kombiniert, wobei eine Zuleitung der Detektordaten von solchen Modulen ohne Funkmodul zu solchen mit einem Funkmodul vorgesehen ist. Beispielswiese können diese abwechselnd nebeneinander angeordnet sein. Dies erhöht zwar die durch ein Funkmodul zu übertragende Datenmenge, jedoch könnten zumindest teilweise eine Kostenersparnis eintreten, da nicht in jedem Detektormodul ein Funkmodul vorgesehen sein muss.

Alle Ausgestaltungsvarianten, die zuvor im Rahmen der erfindungsgemäßen Detektormoduls beschrieben sind, können entsprechend auch in dem Röntgendetektor ausgeführt sein. Die im Hinblick auf das Detektormodul erfolgte Beschreibung und die zuvor beschriebenen Vorteile des Detektormoduls können entsprechend auch auf den erfindungsgemäßen Röntgendetektor übertragen werden.

Der Röntgendetektor kann darüber hinaus weitere Komponenten, beispielsweise ein Gehäuse aufweisen, welches die Module zumindest teilweise umschließt.

Die Erfindung betrifft weiterhin ein medizinisches Bildgebungsgerät umfassend ein Detektormodul oder einen Röntgendetektor gemäß einer der zuvor beschriebenen Varianten.

In Gegenüberstellung zu dem Detektormodul bzw. des Röntgendetektors umfassend ein solches Detektormodul umfasst das medizinische Bildgebungsgerät eine Röntgenquelle, welche ausgebildet ist, das Detektormodul bzw. den Röntgendetektor entlang der Strahleneinfallsrichtung mit Röntgenstrahlung zu belichten.

Für die Aufnahme des Röntgenbilddatensatzes kann dann insbesondere zwischen Röntgenquelle und Detektormodul bzw. Röntgendetektor ein abzubildendes Objekt platziert und mittels der Röntgenquelle durchstrahlt werden.

Insbesondere kann das medizinische Bildgebungsgerät als Computertomographie-System ausgebildet sein. Es kann aber auch beispielsweise als C-Bogen-Röntgengerät und/oder Dyna-CT oder auch als anderweitiges röntgenbasiertes Bildgebungsgerät ausgebildet sein.

Alle Ausgestaltungsvarianten, die zuvor im Rahmen des erfindungsgemäßen Detektormodul bzw. Röntgendetektors beschrieben sind, können entsprechend auch in dem medizinischen Bildgebungsgerät ausgeführt sein. Die im Hinblick auf das Detektormodul bzw. den Röntgendetektor erfolgte Beschreibung und die zuvor beschriebenen Vorteile können entsprechend auch auf das erfindungsgemäße medizinische Bildgebungsgerät übertragen werden.

Gemäß einer Ausbildungsvariante ist das medizinische Bildgebungsgerät als Computertomographiegerät ausgebildet, wobei das zumindest eine Detektormodul bzw. der Röntgendetektor auf dem rotierenden Teil und zumindest eine Empfängereinheit,

welche mit dem Funkmodul für die drahtlose Datenübertragung zusammenwirkt, auf einem stationären Teil des Computertomographiegeräts angeordnet ist.

Die Empfängereinheit umfasst zumindest eine Antenne, welche erlaubt, die vom Funkmodul versendeten Detektordaten zu empfangen und einen Schaltkreis, welche eine Verarbeitung und Weiterleitung der Detektordaten erlaubt. Die Empfängereinheit und das Funkmodul wirken bei der drahtlosen Datenübertragung zusammen und sind aufeinander abgestimmt, so dass die Datenübertragung ermöglicht ist. Eine Platzierung der Empfängereinheit auf dem stationären Teil des CT-Geräts ist vorteilhaft, da hierdurch eine relativ geringe und außerdem definierte Distanz für die Datenübertragung zu überwinden ist. Eine Anordnung außerhalb des CT-Geräts umfasst zum einen größere Übertragungsdistanzen und zum anderen ggf. von Anwendungsfall zu Anwendungsfall unterschiedliche Bedingungen, die berücksichtigt werden müssen.

Gemäß einer Ausbildungsvariante des zuvor beschriebenen Computertomographiegeräts umfasst das Bildgebungsgerät eine Mehrzahl an Empfängereinheiten. Dies kann ggf. eine parallele Datenübertragung an die Mehrzahl an Empfängereinheiten erlauben. Weiterhin ist auch denkbar, dass vorteilhaft jeweils die Empfängereinheit für die Übertragung genutzt wird, welche bei Funkübertragung in optimaler Lage zu einem Funkmodul vorliegt.

Weiterhin können die Empfängereinheiten in verschiedenen Anordnungen auf dem Stator angebracht werden, um jeweils optimale und/oder alternierende Empfangssituationen zu erreichen. Beispiele hierfür sind eine ringförmige Anordnung auf dem stationären Teil um eine Rotationsachse des Computertomographie-Geräts oder eine Anordnung als Gruppe am Stator.

Im Rahmen der Erfindung können außerdem Merkmale, welche in Bezug auf unterschiedliche Ausführungsformen der Erfindung beschrieben sind, zu weiteren Ausführungsformen der Erfindung kombiniert werden. Neben den in dieser Anmeldung ausdrücklich beschriebenen Ausführungsformen der Erfindung sind vielfältige weitere Ausführungsformen der Erfindung denkbar, zu denen der Fachmann gelangen kann, ohne den Bereich der Erfindung zu verlassen, der durch die Ansprüche vorgegeben ist.

Die Verwendung der unbestimmten Artikel "ein" bzw. "eine" schließt nicht aus, dass das betroffene Merkmal auch mehrfach vorhanden sein kann. Die Verwendung des Ausdrucks "aufweisen" schließt nicht aus, dass die mittels des Ausdrucks "aufweisen" verknüpften Begriffe identisch sein können. Beispielsweise weist das medizinische Bildgebungsgerät das medizinische Bildgebungsgerät auf. Die Verwendung des Ausdrucks "Einheit" schließt nicht aus, dass der Gegenstand, auf den sich der Ausdruck "Einheit" bezieht, mehrere Komponenten aufweisen kann, die räumlich voneinander separiert sind.

Im Folgenden wird die Erfindung anhand von beispielhaften Ausführungsformen unter Hinweis auf die beigefügten Figuren erläutert. Die Darstellung in den Figuren ist schematisch, stark vereinfacht und nicht zwingend maßstabsgetreu. Es zeigen:
Fig. 1 ein Detektormodul in einem schematischen Querschnitt gemäß einer Ausbildungsvariante,
Fig. 2 bis Fig. 5 Detailansichten einer Stapelanordnung eines Detektormoduls gemäß verschiedener Ausbildungsvarianten,
Fig. 6 schematisch das Zusammenwirken einzelner Komponenten des Detektormoduls, und
Fig. 7 eine schematische Darstellung eines medizinischen Bildgebungsgeräts.

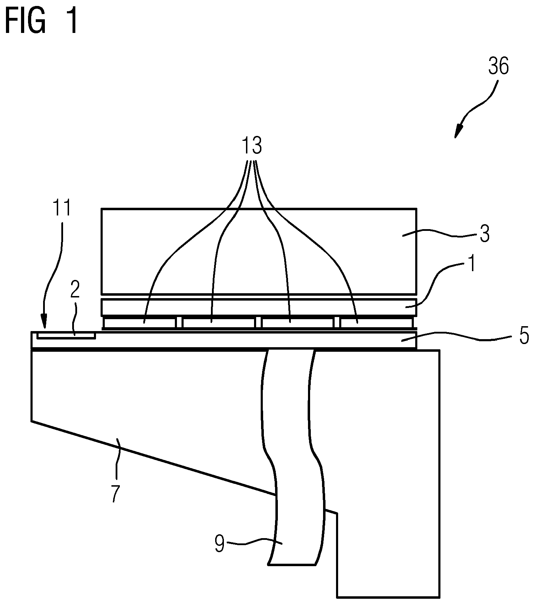

Fig. 1 zeigt ein Detektormodul in einem schematischen Querschnitt gemäß einer Ausbildungsvariante.

Das Detektormodul weist eine Sensoreinheit 1 auf, ausgebildet zur Konvertierung von eintreffender Röntgenstrahlung in elektrische Signale. Weiterhin weist es zumindest eine Ausleseeinheit 13, ausgebildet zur Auslese der elektrischen Signale von der Sensoreinheit 1. In der gezeigten Variante weist das Detektormodul zumindest vier Ausleseeinheiten 13 auf. Es könnten jedoch auch eine andere Anzahl sein, beispielsweise 8 sein oder auch nur eine. Weiterhin weist das Detektormodul ein Funkmodul 2 mit einem Funkschaltkreis 21 auf, welches ausgebildet ist, die ausgelesenen elektrischen Signale mittels eines Verfahrens der drahtlosen Datenübertragung zu versenden. Weiterhin weist das Detektormodul eine in Stapelanordnung zur Sensoreinheit 1 angeordnete Elektronikeinheit 5 auf, wobei der Funkschaltkreis 21 des Funkmoduls 2 zumindest teilweise in ein Einbettungsmaterial der Elektronikeinheit 5 eingebettet ist. Hier nicht explizit gezeigt, ist die erfindungsgemäße zumindest eine elektrisch leitende Verbindung 17 zur Weiterleitung der ausgelesenen elektrischen Signale von der zumindest einen Ausleseeinheit 13 an das Funkmodul 2, welche die Elektronikeinheit 5 aufweist.

Die Sensoreinheit kann dabei als eine direkt-konvertierende Sensoreinheit aufweisend ein geeignetes Konvertermaterial wie beispielsweise CdTe, CZT, CdZnTeSe, CdTeSe, CdMnTe, InP, TlBr2, HgI2, GaAs ausgebildet sein. Alternativ umfasst die Sensoreinheit 1 ein indirekt-konvertierendes Konvertermaterial 25, insbesondere ein Szintillator, und zumindest ein damit gekoppeltes Photodiodenarray 29.

Die Sensoreinheit 1 liegt erfindungsgemäß in Stapelanordnung mit der Elektronikeinheit 5 vor. In der hier gezeigten Ausführung sind weiterhin die Ausleseeinheiten 13 in Stapelanordnung zwischen der Sensoreinheit 1 und der Elektronikeinheit 5 und direkt anschließend an die Sensoreinheit angeordnet und mit dieser, beispielsweise über sogenannte bump bonds oder eine Leitklebeverbindung, für eine Signalübertragung verbunden. Es kann jedoch auch andere Anordnungen geben. Insbesondere kann die Elektronikeinheit 5 auch zwischen den Ausleseeinheiten 13 und der Sensoreinheit 1 angeordnet sein, wobei die Ausleseeinheiten dann mittels entsprechend in der Elektronikeinheit 5 ausgebildeten elektrisch leitenden Verbindungen mit der Sensoreinheit 1 verbunden sind. In einer weiteren Alternative können die Ausleseeinheiten 13 auch in das Einbettungsmaterial 15 der Elektronikeinheit 5 eingebettet sein. Die Ausleseeinheiten 13 können beispielsweise als ASIC ausgebildet sein.

Die Elektronikeinheit 5 umfasst in der Regel ein nicht leitendes Einbettungsmaterial, insbesondere ein Polymer, beispielsweise ein Plastikmaterial oder ein Epoxidharz, und eine oder mehrere elektrisch leitende Verbindungen, welche eingebettet in das Einbettungsmaterial oder auf einer Oberfläche des Einbettungsmaterial ausgebildet sind. Außerdem ist der Funkschaltkreis des Funkmoduls 2, umfassend zumindest den Funkschaltkreis und eine Funkantenne bzw. ein Funkantennenarray (siehe auch folgende Figuren), erfindungsgemäß in das Einbettungsmaterial der Elektronikeinheit 5 eingebettet. Das Funkmodul 2 ist ausgebildet, die Detektordaten mittels eines Verfahrens der drahtlosen Datenübertragung, d.h. mittels einer Funktechnik zu versenden. Das Funkmodul als Sender arbeitet dabei in der Regel mit einem außerhalb des Röntgendetektors platzierten weiteren Funkmodul als Empfänger zusammen, so dass die Detektordaten von dem Detektormodul zu dem außerhalb des Röntgendetektors platzierten, weiteren Empfänger-Funkmodul übertragen werden können. Es kann auch Ausbildungen geben, wobei das Funkmodul 2 des Detektormoduls außerdem als Empfänger ausgebildet sein kann, um Funksignale, beispielsweise umfassend Ansteuerungssignale für das Detektormodul, von einem Sender, welcher außerhalb des Röntgendetektors platziert ist, zu empfangen.

Die Elektronikeinheit 5 mit dem Funkschaltkreis kann insbesondere besonders vorteilhaft mit sogenannten Wafer-Level-Packaging-Methoden, bevorzugt einer Methode des Fan-out Wafer-Level Packaging, oder Panel-Level Packaging-Methoden hergestellt sein. Vorteilhaft können hier in kostengünstiger Weise und parallelisiert in größerer Anzahl Elektronikeinheiten mit bereits integrierten Funkschaltkreisen bereitgestellt werden.

Erfindungsgemäß weist die Elektronikeinheit 5 außerdem einen Flächenbereich auf, welcher in einer Richtung senkrecht zur Stapelrichtung der Stapelanordnung über die flächige Ausdehnung der Sensoreinheit übersteht, wobei das Funkmodul 2 in dem überstehenden Flächenbereich angeordnet ist. Dadurch wird eine drahtlose Datenübertragung nicht durch die Sensoreinheit oder andere Komponenten des Detektormoduls gestört. In vorteilhafter Weise kann außerdem vermieden werden, dass der Funkschaltkreis zu einem Wärmeeintrag in die Sensoreinheit führt, was zu Einbußen bei einer Bildqualität führen könnte.

In der hier gezeigten Variante weist das Detektormodul weitere Komponenten auf, wie eine Trägereinheit 7, welche außerdem als Kühlkörper wirken könnte. Außerdem ist ein Streustrahlengitter 3 vorgesehen, welche in Strahleneinfallsrichtung vor der Sensoreinheit angeordnet ist. Auch sind hier elektrische Leitungen 9 vorgesehen, welche beispielsweise eine Betriebsspannung oder, sofern nicht per Funk übertragen, Steuersignale für die Ausleseeinheiten oder für das Funkmodul 2, zuführen können.

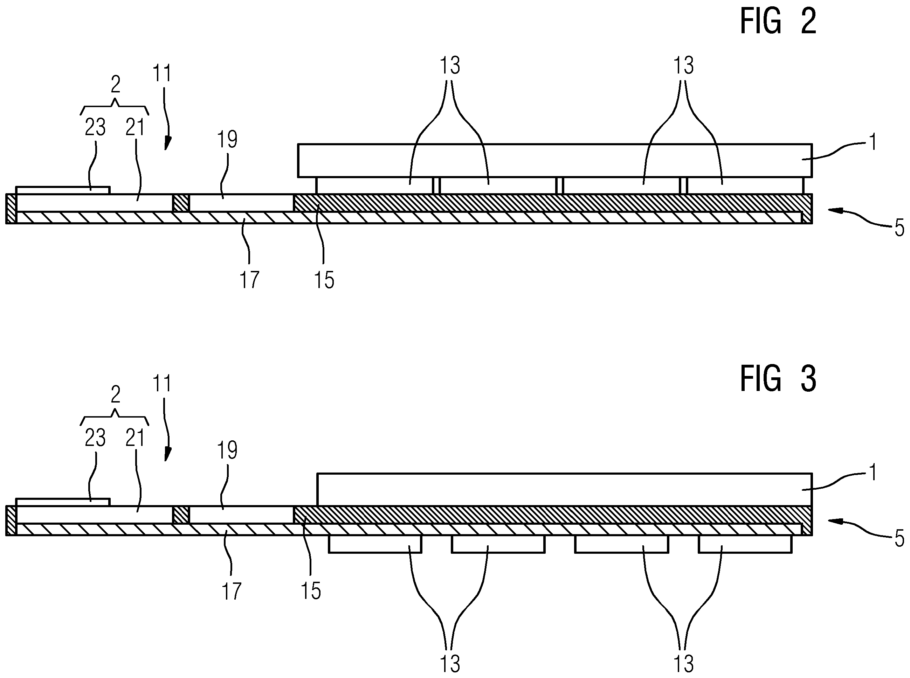

Fig. 2 bis Fig. 5 zeigen Detailansichten einer Stapelanordnung eines Detektormoduls gemäß verschiedener Ausbildungsvarianten.

So zeigt Fig. 2 wie in Fig. 1 die Auswerteeinheiten 13 in Stapelanordnung und anschließend an die Sensoreinheit 1 angeordnet. Die Auswerteeinheiten 13 liegen also in der Stapelanordnung zwischen der Sensoreinheit 1 und der Elektronikeinheit 5 vor. Weiterhin ist in das Einbettungsmaterial 15 der Elektronikeinheit 5 eine Umverdrahtungslage 17 integriert, welche die elektrisch leitenden Verbindungen zwischen den Einheiten verwirklicht. Die Umverdrahtungslage kann beispielsweise derart ausgebildet sein, dass eine Verdrahtung auf einem Substrat ausgebildet wird, welches gemeinsam mit den Schaltkreisen zu dem Package der Elektronikeinheit 5 durch das Einbettungsmaterial vergossen wird. Sie kann auch anders ausgeführt sein. Neben der Verdrahtungslage können weitere elektrisch leitende Verbindungen auch in vertikaler Richtung ausgebildet sein, welche die Kontaktierung der Ausleseeinheiten ermöglichen. Der Funkschaltkreis kann beispielsweise als ASIC ausgebildet sein. Die Funkantenne 23 des Funkmoduls 2 umfasst zumindest einen auf die Oberfläche der Elektronikeinheit 5 aufgebrachten Draht. Dieser kann beispielsweise lithographisch, mittels einer Sputtertechnik oder mittels eines Verfahrens des Leitungsdrucks aufgebracht sein. Die Funkantenne 23 ist in vorteilhafter Weise als Funkantennenarray ausgebildet.

Gemäß einer weiteren Ausbildungsvariante umfasst das Detektormodul weiterhin einen separaten Steuerungsschaltkreis 19, welcher ausgebildet ist, die Ausleseeinheiten 13 und das Funkmodul 2 anzusteuern. In vorteilhaft günstigen Ausbildungsvarianten ist der Steuerschaltkreis dabei insbesondere als FPGA ausgebildet. Es kann jedoch auch andere Ausbildungsvarianten geben, beispielsweise als ASIC. Insbesondere kann es auch Ausbildungen geben, bei denen der Steuerschaltkreis 19 und der Funkschaltkreis 21 als ein gemeinsamer Schaltkreis ausgeführt sind. Ebenso kann eine Übermittlung von Ansteuerungssignalen weiterhin über ein dafür vorgesehenes Kabel erfolgen.

Auch der Steuerungsschaltkreis 19 ist hier in das Einbettungsmaterial 15 der Elektronikeinheit 5 eingebettet, so dass vorteilhaft ein kompakter Aufbau und ein integrales Package aus Funkmodul 2, Steuerschaltkreis 19 und entsprechender Verdrahtung 17 bereitgestellt ist.

Fig. 3 zeigt im Wesentlichen die gleichen Einheiten wie in Fig. 2, wobei jedoch die Anordnung der Ausleseeinheiten 13 verschieden ist. In dieser Variante ist die Elektronikeinheit 5 in der Stapelanordnung insbesondere zwischen der Sensoreinheit 1 und den Ausleseeinheiten 13 angeordnet. Die Elektronikeinheit 5 weist dann entsprechend auch elektrisch leitende Verbindungen auf, welche die Sensoreinheit 1 mit den Ausleseeinheiten 13 verbinden. Die Elektronikeinheit 5 dient hier als sogenannter Interposer und kann hier beispielsweise eine Umverdrahtung verwirklichen von einer insgesamt größeren Sensorfläche der Sensoreinheit 1 auf Ausleseeinheiten 13 mit geringeren Abmessungen, welche kostengünstiger bereitstellbar sind.

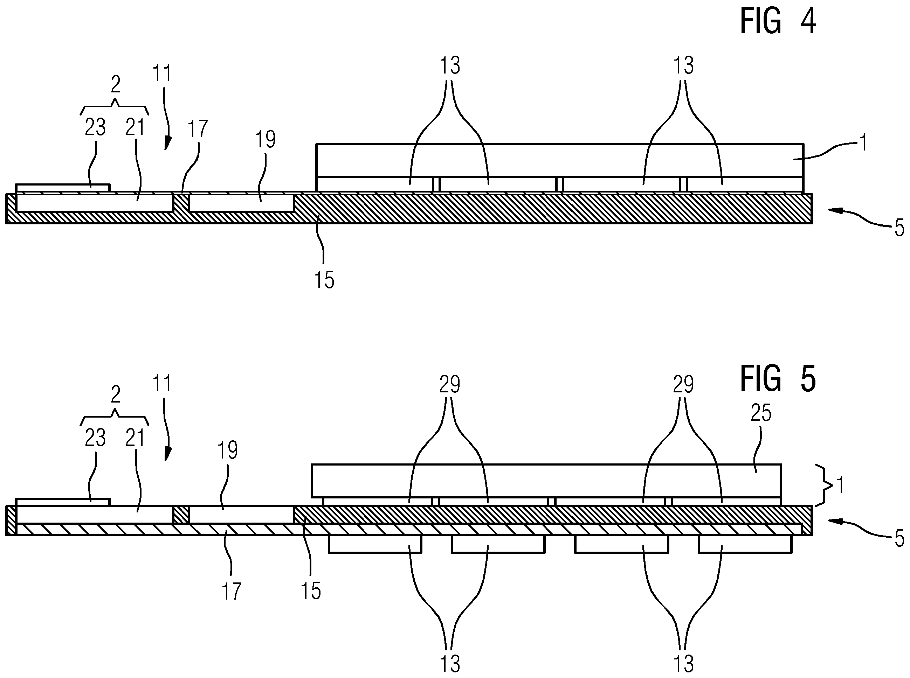

Fig. 4 zeigt im Gegensatz zu Fig. 2 keine in das Einbettungsmaterial der Elektronikeinheit integrierte Umverdrahtungslage, sondern eine, beispielsweise lithographisch, auf eine Oberfläche aufgebrachte Umverdrahtung 17, welche die Einheiten des Detektormoduls für die Signalübertragung entsprechend verbindet. Hierdurch kann in Varianten eine weitere Reduzierung der Bauhöhe erreicht werden.

Fig. 5 zeigt weiterhin im Gegensatz zu Fig. 2 explizit die Ausbildung der Sensoreinheit 1 als indirekt-konvertierende Sensoreinheit 1 umfassend einen Szintillator 25 und an diesen angekoppelte Photodiodenarrays 29.

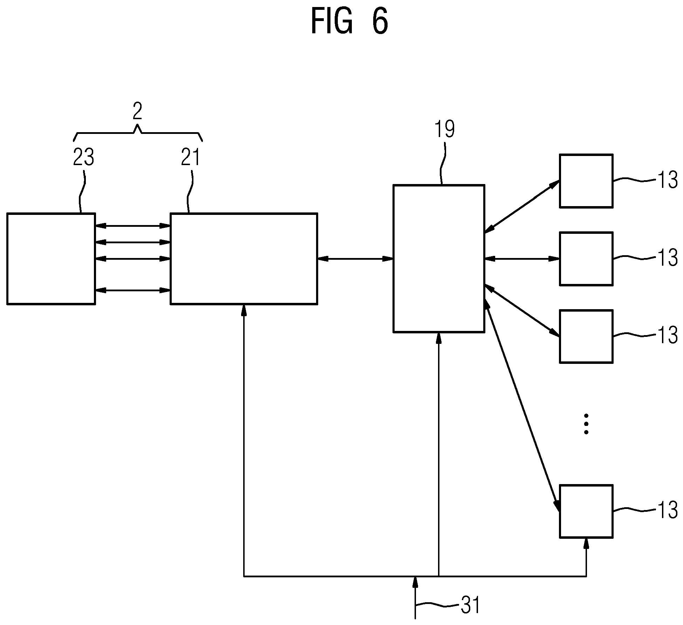

Fig. 6 zeigt schematisch noch einmal das Zusammenwirken der einzelnen Komponenten des Detektormoduls.

Der Steuerungsschaltkreis 19 ist ausgebildet die Ausleseeinheiten 13 zumindest für die Auslese der Detektordaten und den Funkschaltkreis 21 des Funkmoduls 2 anzusteuern. Für die Datenübertragung dient dann ein Antennenarray 23, über welche die Detektordaten an einen entsprechend abgestimmten Empfänger, welcher außerhalb des Detektors angeordnet ist, versendet werden. Für den Betrieb der Ausleseeinheiten 13, des Steuerschaltkreises 19 und des Funkschaltkreises 21 ist außerdem die Zuführung einer Betriebsspannung 31 vorgesehen.

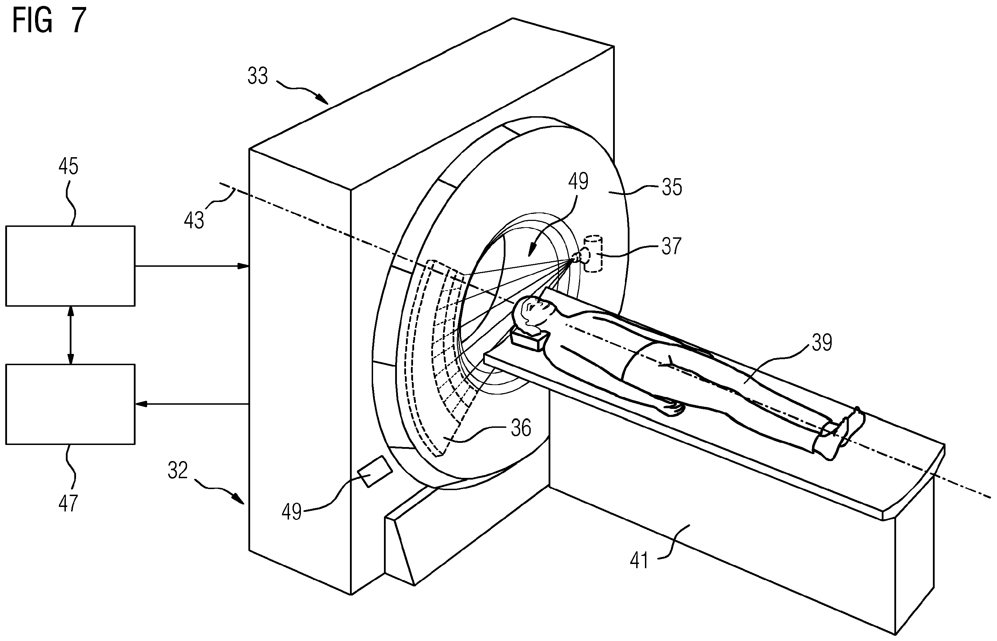

Fig. 7 zeigt weiterhin eine beispielhafte Ausführungsform eines medizinischen Bildgebungsgeräts 32 mit einem Röntgendetektor 36 und einer Röntgenquelle 37 in Gegenüberstellung zum Röntgendetektor 36. Die Röntgenquelle 37 ist ausgebildet, den Röntgendetektor 36 entlang einer Strahleneinfallsrichtung mit Röntgenstrahlung zu belichten. Das gezeigte medizinische Bildgebungsgerät 32 ist insbesondere als CT-Gerät ausgebildet. Das CT-Gerät beinhaltet eine Gantry 33 mit einem Rotor 35. Der Rotor 35 umfasst die Röntgenquelle 37 und den Röntgendetektor 36. Der Rotor 35 ist um die Rotationsachse 43 drehbar. Das Untersuchungsobjekt 39, hier ein Patient, ist auf der Patientenliege 41 gelagert und ist entlang der Rotationsachse 43 durch die Gantry 33 bewegbar. Zur Steuerung des Computertomographie-Systems und zur Berechnung von Schnittbilder bzw. Volumenbildern des Objekts wird eine Recheneinheit 45 verwendet. Die Recheneinheit 45 in Form eines Computersystems ist ausgebildet, Röntgenbilddaten basierend auf den Daten des Röntgendetektors 36 des CT-Geräts zu rekonstruieren. Ein weiteres Computersystem dient als Bedienerkonsole 47. Die auf der Bedienerkonsole 47 installierte Software ermöglicht es dem Bediener, den Betrieb des CT-Geräts zu steuern, wie z. B. die Auswahl eines Protokolls, das Starten des Scannens, usw. Die Bedienkonsole 47 kann auch als ein Computersystem ausgebildet sein.

Der Röntgendetektor 36 umfasst insbesondere eine Mehrzahl an Detektormodulen gemäß einer der zuvor beschriebenen Varianten, jeweils umfassend ein Funkmodul 2.

Die Mehrzahl an Detektormodulen kann insbesondere nebeneinander in dem Röntgendetektor 36 angeordnet sein, so dass die Sensoreinheiten 1 insgesamt als größere Detektionsfläche zusammenwirken. Vorteilhaft weist jedes Detektormodul des Röntgendetektors 36 ein Funkmodul auf, so dass die in einem Detektormodul entstehenden Detektordaten unmittelbar an die jeweilige in diesem Detektormodul vorliegende Funkeinheit übergeben und ohne weitere Zwischenschritte und -wege direkt übertragen werden können. Es wäre auch denkbar, dass ein erfindungsgemäßer Röntgendetektor Detektormodule mit einem Funkmodul und solche ohne ein Funkmodul kombiniert, wobei eine Zuleitung der Detektordaten von solchen Modulen ohne Funkmodul zu solchen mit einem Funkmodul vorgesehen ist.

Das CT-Gerät weist außerdem zumindest eine Empfängereinheit 49 auf dem stationären Teil auf, welche mit den Funkmodulen 2 für die drahtlose Datenübertragung zusammenwirkt.

Die Empfängereinheit 49 umfasst zumindest eine Empfänger-Antenne, welche erlaubt, die von einem Funkmodul 2 versendeten Detektordaten zu empfangen und einen Schaltkreis, welche eine Verarbeitung und Weiterleitung der Detektordaten erlaubt. Eine Platzierung der Empfängereinheit 49 auf dem stationären Teil des CT-Geräts ist vorteilhaft, da hierdurch eine relativ geringe und außerdem definierte Distanz für die Datenübertragung zu überwinden ist. Eine Anordnung außerhalb des CT-Geräts umfasst zum einen größere Übertragungsdistanzen und ggf. von Anwendungsfall zu Anwendungsfall unterschiedliche Bedingungen, die berücksichtigt werden müssen. Eine derartige Platzierung ist aber ebenfalls möglich.

In vorteilhaften Ausbildungsvarianten umfasst das CT-Gerät eine Mehrzahl an Empfängereinheiten 49. Diese können in verschiedenen Anordnungen auf dem Stator angebracht werden, um jeweils optimale und/oder alternierende Empfangssituationen zu erreichen. Beispiele hierfür sind eine ringförmige Anordnung auf dem stationären Teil um eine Rotationsachse des CT-Geräts oder eine Anordnung als Gruppe am Stator.

## Patentansprüche

1. Detektormodul für einen Röntgendetektor (36) aufweisend
- eine Sensoreinheit (1), ausgebildet zur Konvertierung von eintreffender Röntgenstrahlung in elektrische Signale, und aufweisend eine Vielzahl an Pixelelementen,
- zumindest eine Ausleseeinheit (13) in Stapelanordnung mit der Sensoreinheit (1), ausgebildet zur Auslese der elektrischen Signale von der Sensoreinheit (1), wobei jedes Pixelelement mit einem zugeordneten Auswertepixelelement der Ausleseeinheit (13) verbunden ist,
- ein Funkmodul (2) mit einem Funkschaltkreis (21), welches ausgebildet ist, die ausgelesenen elektrischen Signale mittels eines Verfahrens der drahtlosen Datenübertragung zu versenden, und
- eine in Stapelanordnung zur Sensoreinheit (1) angeordnete Elektronikeinheit (5) aufweisend zumindest eine elektrisch leitende Verbindung (17) zur Weiterleitung der ausgelesenen elektrischen Signale von der zumindest einen Ausleseeinheit (13) an das Funkmodul (2), **dadurch gekennzeichnet, dass** der Funkschaltkreis (21) des Funkmoduls (2) zumindest teilweise in ein Einbettungsmaterial (15) der Elektronikeinheit (5) eingebettet ist, wobei die Elektronikeinheit (5) einen Flächenbereich (11) aufweist, welcher in einer Richtung senkrecht zur Stapelrichtung über die flächige Ausdehnung der Sensoreinheit (1) übersteht, und wobei das Funkmodul (2) in dem überstehenden Flächenbereich (11) angeordnet ist.

2. Detektormodul nach Anspruch 1, wobei die zumindest eine Ausleseeinheit (13) zumindest teilweise in das Einbettungsmaterial (15) der Elektronikeinheit (5) eingebettet ist.

3. Detektormodul nach einem der vorangehenden Ansprüche, wobei das Funkmodul (2) zumindest eine Funkantenne (23) aufweist und wobei die Funkantenne (23) einen auf das Einbettungsmaterial (15) der Elektronikeinheit (5) oder auf einer Oberfläche des Funkschaltkreises (21) aufgebrachten Draht umfasst.

4. Detektormodul nach einem der vorangehenden Ansprüche, außerdem aufweisend einen Steuerungsschaltkreis (19), welcher ausgebildet ist, die zumindest eine Ausleseeinheit (13) und/oder das Funkmodul (2) anzusteuern.

5. Detektormodul nach Anspruch 4, wobei der Steuerungsschaltkreis (19) zumindest teilweise in das Einbettungsmaterial (15) der Elektronikeinheit (5) eingebettet ist.

6. Detektormodul nach einem der vorangehenden Ansprüche, wobei die Elektronikeinheit (5) mittels einer Methode des Fan-out Wafer-Level Packaging oder Panel-Level Packaging hergestellt ist.

7. Detektormodul nach einem der vorangehenden Ansprüche, wobei die zumindest eine elektrisch leitende Verbindung (17) zur Signalübertragung zwischen der zumindest einen Ausleseeinheit (13) und dem Funkmodul (2) in die Elektronikeinheit (5) integriert oder auf dem Einbettungsmaterial (15) der Elektronikeinheit (5) aufgebracht ist.

8. Detektormodul nach einem der vorangehenden Ansprüche, wobei die Elektronikeinheit (5) in der Stapelanordnung zwischen der Sensoreinheit (1) und der zumindest einen Ausleseeinheit (13) angeordnet ist.

9. Detektormodul nach einem der vorangehenden Ansprüche, wobei die Sensoreinheit (1) als direktkonvertierende Sensoreinheit ausgebildet ist oder ein Szintillatorelement (25) und zumindest ein Photodiodenarray (29) umfasst.

10. Röntgendetektor (36) umfassend eine Mehrzahl an Detektormodulen nach einem der vorangehenden Ansprüche.

11. Medizinisches Bildgebungsgerät (32) umfassend zumindest ein Detektormodul nach einem der Ansprüche 1 bis 9 oder einen Röntgendetektor (36) nach Anspruch 10 und eine in Gegenüberstellung dazu angeordnete Röntgenquelle (37).

12. Medizinisches Bildgebungsgerät (32) nach Anspruch 11, wobei das medizinische Bildgebungsgerät (32) als Computertomographiegerät ausgebildet ist und wobei das zumindest eine Detektormodul auf einem rotierenden Teil und zumindest eine Empfängereinheit (49), welche mit dem Funkmodul (2) für die drahtlose Datenübertragung zusammenwirkt, auf einem stationären Teil des Computertomographiegeräts angeordnet ist.

13. Medizinisches Bildgebungsgerät (32) nach Anspruch 12, umfassend eine Mehrzahl an Empfängereinheiten (49), welche beabstandet zueinander auf dem stationären Teil radial um eine Rotationsachse (43) des Computertomographie-Geräts oder als Gruppe auf dem stationären Teil des Computertomographie-Geräts angeordnet sind.

## Claims

1. Detector module for an X-ray detector (36) having
- a sensor unit (1) configured to convert incoming X-rays into electrical signals, and having a plurality of pixel elements,
- at least one readout unit (13) in a stacked arrangement with the sensor unit (1), configured to read out the electrical signals from the sensor unit (1), wherein each pixel element is connected to an associated evaluation pixel element of the readout unit (13),
- a radio module (2) with a radio circuit (21) which is configured to transmit the read-out electrical signals by means of a wireless data transmission method, and
- an electronic unit (5) arranged in a stacked arrangement with respect to the sensor unit (1) having at least one electrically conductive connection (17) for forwarding the read-out electrical signals from the at least one read-out unit (13) to the radio module (2),
**characterised in that**
the radio circuit (21) of the radio module (2) is at least partially embedded in an embedding material (15) of the electronic unit (5), wherein the electronic unit (5) has a surface area (11) which protrudes in a direction perpendicular to the stacking direction beyond the planar extension of the sensor unit (1), and wherein the radio module (2) is arranged in the protruding surface area (11).

2. Detector module according to claim 1, wherein the at least one read-out unit (13) is at least partially embedded in the embedding material (15) of the electronic unit (5).

3. Detector module according to one of the preceding claims, wherein the radio module (2) has at least one radio antenna (23) and wherein the radio antenna (23) comprises a wire applied to a surface of the embedding material (15) of the electronic unit (5) or to a surface of the radio circuit (21).

4. Detector module according to one of the preceding claims, also comprising a control circuit (19), which is configured to control the at least one readout unit (13) and/or the radio module (2).

5. Detector module according to claim 4, wherein the control circuit (19) is at least partially embedded in an embedding material (15) of the electronic unit (5).

6. Detector module according to one of the preceding claims, wherein the electronic unit (5) is manufactured using a fan-out wafer-level packaging or panel-level packaging method.

7. Detector module according to one of the preceding claims, wherein the at least one electrically conductive connection (17) for signal transmission between the at least one read-out unit (13) and the radio module (2) is integrated in the electronic unit (5) or applied to the embedding material (15) of the electronic unit (5).

8. Detector module according to one of the preceding claims, wherein the electronic unit (5) is arranged in the stack arrangement between the sensor unit (1) and the at least one readout unit (13).

9. Detector module according to one of the preceding claims, wherein the sensor unit (1) is configured as a direct-conversion sensor unit or a scintillator element (25) and at least one photodiode array (29).

10. X-ray detector (36) comprising a plurality of detector modules according to one of the preceding claims.

11. Medical imaging device (32) comprising at least one detector module according to one of claims 1 to 9 or an X-ray detector (36) according to claim 10 and an X-ray source (37) arranged in opposition thereto.

12. Medical imaging device (32) according to claim 11, wherein the medical imaging device (32) is configured as a computed tomography device, and wherein the at least one detector module is arranged on a rotating part and at least one receiver unit (49), which acts together with the radio module (2) for the wireless data transmission, is arranged on a stationary part of the computed tomography device.

13. Medical imaging device (32) according to claim 12, comprising a plurality of receiver units (49), which are arranged spaced apart from each other on the stationary part radially about an axis of rotation (43) of the computed tomography device or as a group on the stationary part of the computed tomography device.

## Revendications

1. Module détecteur pour un détecteur (36) de rayons X comportant
- une unité (1) de capteur, constituée pour la transformation de rayonnement X incident en des signaux électriques, et comportant une pluralité d'éléments de pixel,
- au moins une unité (13) de lecture suivant un agencement empilé avec l'unité (1) de capteur, constituée pour la lecture des signaux électriques de l'unité (1) de capteur, dans lequel chaque élément de pixel est relié à un élément de pixel d'évaluation associé de l'unité (13) de lecture,
- un module (2) radio ayant un circuit (21) radio, qui est constitué pour envoyer les signaux électriques lus au moyen d'un procédé de transmission de données sans fil, et
- une unité (5) électronique disposée en un agencement empilé par rapport à l'unité (1) de capteur et comprenant au moins une liaison (17) conductrice de l'électricité pour l'acheminement des signaux électriques lus de la au moins une unité (13) de lecture au module (2) radio,
**caractérisé en ce que** le circuit (21) radio du module (2) radio est enrobé au moins en partie dans une matière (15) d'enrobage de l'unité (5) électronique, dans lequel l'unité (5) électronique a une partie (11) de surface qui, dans une direction perpendiculaire à la direction d'empilement, dépasse de l'étendue en surface de l'unité (1) de capteur, et dans lequel le module (2) radio est disposé dans la partie (11) de surface en dépassement.

2. Module détecteur suivant la revendication 1, dans lequel la au moins une unité (13) de lecture est enrobée au moins en partie dans la matière (15) d'enrobage de l'unité (5) électronique.

3. Module détecteur suivant l'une des revendications précédentes, dans lequel le module (2) radio a au moins une antenne (23) radio et dans lequel l'antenne (23) radio comprend un fil déposé sur la matière (15) d'enrobage de l'unité (5) électronique ou sur une surface du circuit (21) radio.

4. Module détecteur suivant l'une des revendications précédentes, comportant en outre un circuit (19) de commande, qui est constitué pour commander la au moins une unité (13) de lecture et/ou le module (2) radio.

5. Module détecteur suivant la revendication 4, dans lequel le circuit (19) de commande est enrobé au moins en partie dans la matière (15) d'enrobage de l'unité (5) électronique.

6. Module détecteur suivant l'une des revendications précédentes, dans lequel l'unité (5) électronique est fabriquée au moyen d'un procédé du fan-out wafer-level packaging ou panel-level packaging.

7. Module détecteur suivant l'une des revendications précédentes, dans lequel la au moins une liaison (17) conductrice de l'électricité est, pour la transmission du signal entre la au moins une unité (13) de lecture et le module (2) radio, intégrée à l'unité (5) électronique ou est déposée sur la matière (15) d'enrobage de l'unité (5) électronique.

8. Module détecteur suivant l'une des revendications précédentes, dans lequel l'unité (5) électronique est, dans l'agencement en empilement, disposée entre l'unité (1) de capteur et la au moins une unité (13) de lecture.

9. Module détecteur suivant l'une des revendications précédentes, dans lequel l'unité (1) de capteur est constituée en une unité de capteur à transformation directe ou comprend un élément (25) de scintillateur et au moins un réseau (29) de photodiodes.

10. Détecteur (36) de rayons X comprenant une pluralité de modules détecteurs suivant l'une des revendications précédentes.

11. Appareil (32) d'imagerie médicale comprenant au moins un module détecteur suivant l'une des revendications 1 à 9 ou un détecteur (36) de rayons X suivant la revendication 10 et une source (37) de rayons X disposée en opposition à celui-ci.

12. Appareil (32) d'imagerie médicale suivant la revendication 11, dans lequel l'appareil (32) d'imagerie médicale est constitué en appareil de tomodensitométrie assisté par ordinateur et dans lequel le au moins un module détecteur est disposé sur une partie tournante et au moins une unité (49) de réception, qui coopère avec le module (2) radio pour la transmission sans fil de données, est montée sur une partie fixe sur l'appareil de tomodensitométrie.

13. Appareil (32) d'imagerie médicale suivant la revendication 12, comprenant une pluralité d'unités (49) de réception, qui sont à distance les unes des autres sur la partie fixe radialement autour d'un axe (43) de rotation de l'appareil de tomodensitométrie assisté par ordinateur ou comme groupe sur la partie fixe de l'appareil de tomodensitométrie assisté par ordinateur.
